(19) **Europäisches Patentamt**
**European Patent Office**
**Office européen des brevets**

(11) **EP 3 160 336 B1**

(12) **FASCICULE DE BREVET EUROPEEN**

(45) Date de publication et mention de la délivrance du brevet:
**16.12.2020 Bulletin 2020/51**

(21) Numéro de dépôt: **15732605.9**

(22) Date de dépôt: **23.06.2015**

(51) Int Cl.:
*A61B 5/024* (2006.01)     *A61B 5/0444* (2006.01)
*A61B 5/04* (2006.01)      *A61B 5/0402* (2006.01)
*A61B 5/00* (2006.01)

(86) Numéro de dépôt international:
**PCT/EP2015/064158**

(87) Numéro de publication internationale:
**WO 2015/197650 (30.12.2015 Gazette 2015/52)**

(54) **DISPOSITIF DE TRAITEMENT DE DONNÉES DE RYTHME CARDIAQUE FOETAL, MÉTHODE ET PROGRAMME D'ORDINATEUR CORRESPONDANT**

VORRICHTUNG ZUR VERARBEITUNG VON FÖTALEN HERZRHYTHMUSDATEN SOWIE ENTSPRECHENDES VERFAHREN UND COMPUTERPROGRAMM

DEVICE FOR PROCESSING FOETAL HEART RHYTHM DATA, AND CORRESPONDING METHOD AND COMPUTER PROGRAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.06.2014 FR 1456107**

(43) Date de publication de la demande:
**03.05.2017 Bulletin 2017/18**

(73) Titulaires:
• **Université de Rennes I**
**35065 Rennes Cedex (FR)**
• **Institut National de la Santé et de la Recherche Médicale**
**75013 Paris 13 (FR)**
• **Centre Hospitalier Universitaire de Pontchaillou**
**35000 Rennes (FR)**

(72) Inventeurs:
• **CARRAULT, Guy**
**F-35510 Cesson Sévigné (FR)**
• **VANDENBROUCKE, Laurent**
**F-35000 Rennes (FR)**
• **PLADYS, Patrick**
**F-35530 Noyal sur Vilaine (FR)**
• **POULAIN, Patrick**
**F-35000 Rennes (FR)**

• **BEUCHEE, Alain**
**F-35200 Rennes (FR)**

(74) Mandataire: **Vidon Brevets & Stratégie**
**16B, rue de Jouanet**
**BP 90333**
**35703 Rennes Cedex 7 (FR)**

(56) Documents cités:
**US-A1- 2004 133 115**

• D SUBTIL ET AL: "Analyse automatisée du rythme cardiaque foetal", EXTRAIT DES MISES À JOUR EN GYNÉCOLOGIE ET OBSTÉTRIQUE, vol. XXIV, 30 novembre 2000 (2000-11-30), XP055170955,
• ANDREA BRAVI ET AL: "Review and classification of variability analysis techniques with clinical applications", BIOMEDICAL ENGINEERING ONLINE, BIOMED CENTRAL LTD, LONDON, GB, vol. 10, no. 1, 10 octobre 2011 (2011-10-10), page 90, XP021110610, ISSN: 1475-925X, DOI: 10.1186/1475-925X-10-90
• DAS S ET AL: "A novel approach for extraction and analysis of variability of baseline", RECENT TRENDS IN INFORMATION SYSTEMS (RETIS), 2011 INTERNATIONAL CONFERENCE ON, IEEE, 21 décembre 2011 (2011-12-21), pages 336-339, XP032112574, DOI: 10.1109/RETIS.2011.6146892 ISBN: 978-1-4577-0790-2

## Description

### 1. Domaine de l'invention

L'invention se rapporte au domaine de l'analyse de rythme cardiaque. L'invention concerne un dispositif d'analyse du rythme cardiaque, plus particulièrement pour l'analyse de paramètres du rythme cardiaque au sein d'un enregistrement. Plus particulièrement, l'invention se rapporte à un dispositif d'analyse d'un rythme cardiaque fœtal, par exemple par cardiotocographie.

### 2. Art Antérieur

La cardiotocographie est une des méthodes de surveillance fœtale de référence. Ses avantages sont multiples : une simplicité d'utilisation, excellente sensibilité, non invasif, etc. Son inconvénient principal est sa faible spécificité (liée à des différences d'appréciation). Cette technique est massivement utilisée en routine clinique.

L'analyse du rythme cardiaque fœtal est utile pour détecter une anomalie pouvant survenir soit lors de la grossesse soit lors de l'accouchement. L'analyse du rythme cardiaque fœtal se base généralement sur quatre critères : le rythme de base, la variabilité de ce rythme de base, les accélérations de ce rythme, et la présence éventuelle de ralentissement.

Des dispositifs ou des systèmes d'analyse du rythme cardiaque fœtal ont été proposés. Ils comprennent, de façon très générale, un moniteur qui reçoit et analyse les signaux de rythme cardiaque fœtal et génère des données présentant un intérêt clinique. Dans certains cas, le moniteur utilise des informations morphologiques dans le signal en plus ou au lieu de déterminer uniquement les informations de fréquence cardiaque. Les données générées par le moniteur peuvent être présentées à des praticiens dans une variété de formes, par exemple sous forme imprimée sur les cartes papier, représenté sur une unité d'affichage (par exemple, un écran d'ordinateur), et transmis par l'intermédiaire de signaux sans fil à un terminal mobile (par exemple, un téléphone intelligent ou un PDA). Le plus souvent, le moniteur comprend un système d'acquisition de données et un analyseur. Le système d'acquisition de données collecte des signaux doppler, par exemple, à travers un ensemble de capteurs posés sur le ventre de la mère. Le premier capteur comprend des cristaux regroupés dans un même capteur qui permettent le recueil du signal cardiaque dans le cas de la cardiotocographie. Le second capteur (tocomètre) mesure la pression et capte les contractions utérines maternelles. Le système d'acquisition de données diffuse, dans l'abdomen, un spectre d'onde sonore. Le signal récupéré par le système d'acquisition est alors traité pour retirer des parasites et l'on obtient un enregistrement du rythme cardiaque fœtal (ERCF) consistant en la restitution de la fréquence cardiaque fœtale à un temps donné.

De tels systèmes permettent, in fine, au praticien de détecter des anomalies. Les anomalies détectées sont de plusieurs types : elles peuvent se rapporter au rythme cardiaque, à ses oscillations ou à une altération de sa périodicité.

Plus particulièrement, de tels systèmes analysent les données du rythme cardiaque fœtal sur des périodes d'enregistrement relativement longues (par exemple trente minutes). À l'issue de cette période temporelle, des résultats d'analyse sont délivrés par l'analyseur. Ils sont basés sur l'intégralité de l'enregistrement. Ces systèmes sont utiles dans certains cas de figure. Cependant, l'information délivrée n'est pas forcément représentative d'une pathologie. En effet, quand bien même l'analyse de ce rythme cardiaque fœtal est automatisée, des erreurs d'interprétation subsistent. Par ailleurs, l'interprétation visuelle du RCF n'est pas fiable et plusieurs études ont démontré l'étendue des variations inter-observateur, mais également intra-observateur. L'analyse informatisée comprend plusieurs paramètres (fréquence cardiaque de référence, nombre d'accélérations et de décélérations, nombre d'épisodes de haute et de basse variation, variation à court terme) calculés sur l'ensemble d'un enregistrement d'une durée variant entre 15 et 45 minutes mais n'a pas démontré d'intérêt pour son utilisation en pratique clinique courante. Les outils plus récents (STAN®) n'ont pas non plus montré de bénéfice et sont invasifs (mise en place d'une électrode de scalp fœtal) et ne sont donc pas utilisables dans tous les cas de figure. Les épisodes de haute et de basse variation sont définis comme toute partie de l'enregistrement où, par rapport à la ligne de base, la variation de l'amplitude sur une minute est supérieure à 32 ms (haute variation) ou inférieure à 30 ms (basse variation) pendant 5 à 6 minutes consécutives. Les épisodes de haute variation sont associés au sommeil actif du fœtus tandis que les épisodes de basse variation sont associés au sommeil calme

La généralisation de l'échographie obstétricale s'est accompagnée de nombreux scores biophysiques d'évaluation d'un état fœtal (score de Manning par exemple) qui n'ont pas fait la preuve de leur utilité en pratique clinique courante. Sur le plan de l'issue de grossesse, l'échographie cervicale dispose d'une bonne valeur prédictive négative sur le risque d'accouchement prématuré mais pas de bonne valeur prédictive positive. Les tests maternels développés dans cette indication (fibronectine fœtale par exemple) possèdent les mêmes inconvénients.

Plus récemment, l'échographie doppler s'est développée, permettant d'évaluer les résistances au sein de certains vaisseaux fœtaux et maternels (artère ombilicale, artère cérébrale moyenne, ductus venosus, artères utérines maternelles). Cette évaluation semble utile dans certaines indications (restriction de croissance, syndrome transfuseur/transfusé dans les grossesses gémellaires) mais n'a pas montré d'intérêt global pour l'obtention de données représentatives d'un état fœtal ou d'une issue de grossesse.

Les autres outils développés ces dernières années (amniocentèse, ponction de sang fœtal) possèdent l'inconvénient d'être invasifs et donc difficilement applicables en pratique clinique courante.

On ne dispose donc pas actuellement d'un moyen simple et non invasif d'obtention de données représentatives d'un état fœtal ou d'une issue de grossesse.

Le document «Analyse automatisée du rythme cardiaque fœtal» (D SUBTIL) décrit l'appareil SONICAID d'Oxford Instruments, et en particulier de l'analyse automatisée du RCF en période anténatale (prénatale). Ce document divulgue un instrument permettant la mesure automatique du RCF, et l'analyse de ce signal pour obtenir de façon automatique le rythme cardiaque de base, le nombre d'épisodes de hautes variations, le nombre d'épisodes de basses variations, la variabilité cardiaque à court terme. Cependant, il ne réalise pas de segmentation des épisodes de haute et de basse variation à visée de calcul de paramètres de variabilité sur ces épisodes.

Le document: « Review and classification of variability analysis » (ANDREA BRAVI) des techniques théoriques d'analyse de la variabilité et leurs applications cliniques. Il ne divulgue pas concrètement un dispositif de traitement de données de rythme cardiaque fœtal, pas plus qu'il ne divulgue des moyens de calculs des moyens de calculs de variabilité.

Le document US 2004/133115 A1 décrit un dispositif permettant de segmenter le signal cardiaque fœtal en épisodes de hautes variations ou de basses variations et donner en sortie le nombre de ces épisodes. Cependant, il ne divulgue pas les aspects liés à l'analyse de la variabilité cardiaque sur chaque épisode segmenté, et à l'analyse des ratios des valeurs représentatives obtenues sur chaque type d'épisode.

Le document « A novel approach for extraction and analysis of variability of baseline » (DAS S) décrit le développement d'un algorithme permettant d'extraire de façon plus efficace et précise la fréquence cardiaque de base (« baseline of FHR ») par mesures de cardiotocographie, pour en déterminer la variabilité. Il ne divulgue pas le module de fractionnement de données et le module de calcul de variabilité.

## 3. Résumé de l'invention

L'invention ne pose pas ces problèmes de l'art antérieur. Plus particulièrement, l'invention apporte une solution simple à la problématique préalablement identifiée.

L'invention est définie par le contenu des revendications.

Selon une implémentation préférée, les différentes étapes des procédés selon l'invention sont mises en œuvre par un ou plusieurs logiciels ou programmes d'ordinateur, comprenant des instructions logicielles destinées à être exécutées par un processeur de données d'un module relais selon l'invention et étant conçu pour commander l'exécution des différentes étapes des procédés.

En conséquence, l'invention vise aussi un programme, susceptible d'être exécuté par un ordinateur ou par un processeur de données, ce programme comportant des instructions pour commander l'exécution des étapes d'un procédé tel que mentionné ci-dessus.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un processeur de données, et comportant des instructions d'un programme tel que mentionné ci-dessus.

Le support d'informations peut être n'importe quelle entité ou dispositif capable de stocker le programme. Par exemple, le support peut comporter un moyen de stockage, tel qu'une ROM, par exemple un CD ROM ou une ROM de circuit microélectronique (clé USB, SSD), ou encore un moyen d'enregistrement magnétique, par exemple une disquette (floppy disc) ou un disque dur.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'informations peut être un circuit intégré dans lequel le programme est incorporé, le circuit étant adapté pour exécuter ou pour être utilisé dans l'exécution du procédé en question.

Selon un mode de réalisation, l'invention est mise en œuvre au moyen de composants logiciels et/ou matériels. Dans cette optique, le terme "module" peut correspondre dans ce document aussi bien à un composant logiciel, qu'à un composant matériel ou à un ensemble de composants matériels et logiciels.

Un composant logiciel correspond à un ou plusieurs programmes d'ordinateur, un ou plusieurs sous-programmes d'un programme, ou de manière plus générale à tout élément d'un programme ou d'un logiciel apte à mettre en œuvre une fonction ou un ensemble de fonctions, selon ce qui est décrit ci-dessous pour le module concerné. Un tel composant logiciel est exécuté par un processeur de données d'une entité physique (terminal, serveur, passerelle, routeur, etc.) et est susceptible d'accéder aux ressources matérielles de cette entité physique (mémoires, supports d'enregistrement, bus de communication, cartes électroniques d'entrées/sorties, interfaces utilisateur, etc.).

De la même manière, un composant matériel correspond à tout élément d'un ensemble matériel (ou hardware) apte à mettre en œuvre une fonction ou un ensemble de fonctions, selon ce qui est décrit ci-dessous pour le module concerné. Il peut s'agir d'un composant matériel programmable ou avec processeur intégré pour l'exécution de logiciel, par exemple un circuit intégré, une carte à puce, une carte à mémoire, une carte électronique pour l'exécution d'un micrologiciel (firmware), etc.

Chaque composante du système précédemment décrit met bien entendu en œuvre ses propres modules logiciels.

Les différents modes de réalisation mentionnés ci-dessus sont combinables entre eux pour la mise en œuvre de l'invention.

**4. Dessins**

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :

- la figure 1 présente fonctionnellement un dispositif tel que proposé ;
- la figure 2 décrit une méthode de mise en œuvre.
- la figure 3 présente techniquement un dispositif tel que proposé;

**5. Description**

5.1. Rappel du principe général

Le principe général de fonctionnement du moniteur objet de la présente technique est de procéder à une évaluation discrétionnaire de l'enregistrement du rythme cardiaque fœtal. Pour ce faire, le dispositif objet de l'invention comprend un module d'analyse. Par ailleurs, on fournit (optionnellement), à ce module d'analyse, des données relatives à des changements d'états comportementaux du fœtus au cours de l'enregistrement, comme décrit postérieurement dans un mode de réalisation. On établit ainsi une corrélation entre les changements comportementaux du fœtus et les données du rythme cardiaque fœtal. Dès lors, le résultat obtenu par le dispositif d'analyse est un résultat relatif. Un tel résultat est avantageux puisqu'il permet de contextualiser une variabilité du rythme cardiaque fœtal.

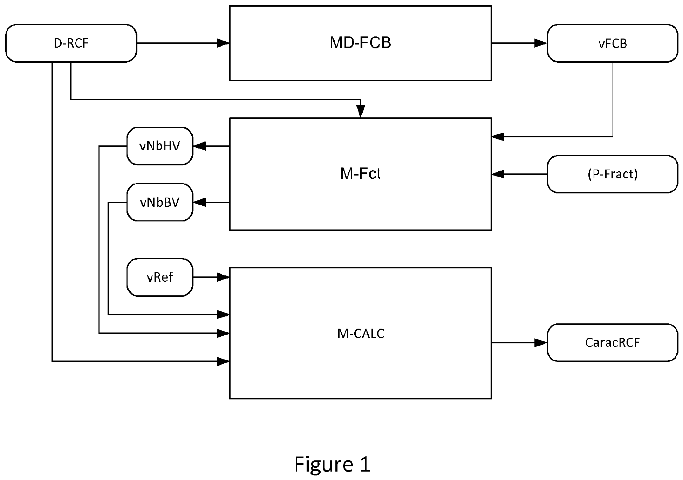

D'une manière générale, en relation avec la figure 1, l'invention se rapporte à un dispositif de traitement de données de rythme cardiaque fœtal (D-RCF) issues d'un enregistrement de rythme cardiaque fœtal, données obtenues par l'intermédiaire d'un dispositif d'enregistrement de rythme cardiaque fœtal. Selon la technique proposée un tel dispositif comprend :

- un module de détermination (MD-FCB) d'une fréquence cardiaque de référence, prenant en entrée les données de rythme cardiaque fœtal (D-RCF) et délivrant une valeur de fréquence cardiaque de référence (vFCB);

- un module de fractionnement (M-Fct) desdites données, en fonction d'un paramètre de fractionnement (P-Fract) et de la valeur de fréquence cardiaque de référence (vFCB) ;
- un module de calcul (M-CALC) d'au moins une caractéristique (CaracRCF) représentative des données de rythme cardiaque fœtal (D-RCF) en fonction de la valeur de fréquence cardiaque de référence (vFCB).

En fonction du mode d'analyse souhaité, le paramètre de fractionnement (P-Fract) des données permet de réaliser d'une part une séparation des données et d'autre part d'établir par la suite une corrélation avec d'autres données externes. Le paramètre de fractionnement (P-Fract) peut être transmis au dispositif soit par l'intermédiaire d'une sélection logicielle effectuée sur le dispositif lui-même (par l'intermédiaire d'une interface homme machine), soit par l'intermédiaire d'un bouton mécanique de sélection d'une mode de fonctionnement du dispositif. Dans un cas général, dans lequel l'ensemble de l'enregistrement est utilisé, le paramètre de fractionnement (P-Fract) n'entraine aucune modification de l'enregistrement. Le module de fractionnement (M-Fct) n'est donc pas mis en œuvre pour effectuer un découpage de l'enregistrement (il peut cependant être mis en œuvre pour obtenir des épisodes de haute variation et des épisodes de basse variation comme cela est exposé par la suite). Dans des cas particuliers, des calculs discrétionnaires peuvent être mis en œuvre (par exemple uniquement sur des épisodes de haute variation ou uniquement sur des épisodes de basse variation). Dans ce cas, le paramètre de fractionnement (P-Fract) est utilisé pour partager les données de l'enregistrement en fonction des calculs discrétionnaires à réaliser.

Le module de fractionnement (M-Fct) comprend par exemple :

- des moyens de détermination d'un nombre d'accélérations des battements cardiaques, les moyens de détermination prenant en entrée les données constitutive de l'enregistrement du rythme cardiaque fœtal et la valeur de fréquence cardiaque de référence (vFCB) et délivrant au moins une valeur représentative d'un nombre d'accélérations ;
- des moyens de détermination d'un nombre d'épisodes de haute variation des battements cardiaques, les moyens de détermination prenant en entrée les données constitutives de l'enregistrement du rythme cardiaque fœtal et délivrant au moins une valeur représentative d'un nombre d'épisodes de haute variation (vNbHV) ;
- des moyens de détermination d'un nombre d'épisodes de basse variation des battements cardiaques, les moyens de détermination prenant en entrée les données constitutives de l'enregistrement du rythme cardiaque fœtal et délivrant au moins une valeur représentative d'un nombre d'épisodes de basse variation (vNbBV) ;

Ces moyens, qui peuvent se présenter sous la forme de modules matériels et logiciels particuliers, permettent en fonction des situations et des besoins de fournir des données qui seront par la suite utilisées pour déterminer d'autres caractéristiques du rythme cardiaque fœtal.

Cet agencement est particulièrement intéressant car il permet de mutualiser des calculs nécessaires à d'autres calculs ultérieurs.

Dans un mode de réalisation général, un tel dispositif s'intègre dans un système complet de surveillance (également appelé moniteur). Un moniteur reçoit et analyse les signaux de rythme cardiaque fœtal et génère des données présentant un intérêt clinique. Les données générées par le moniteur peuvent être présentées à des praticiens dans une variété de formes. Le moniteur comprend un système d'acquisition de données et un dispositif tel que décrit (analyseur).

Le module de calcul (M-CALC) du dispositif comprend

- des moyens de calcul d'une donnée représentative d'un rapport entre la valeur représentative d'un nombre d'épisodes de haute variation (vNbHV) et au moins une valeur de référence (VRef), le rapport étant appelé rapport des épisodes de haute variation (rEHV) ;
- des moyens de transmission d'une donnée représentative d'une évolution dudit rapport des épisodes de haute variation (rEHV).

Ainsi, il est possible d'obtenir un rapport (rEHV) représentatif d'une évolution ou d'une variation des épisodes de haute variation du rythme cardiaque.

De manière complémentaire, les moyens de transmission d'une donnée représentative d'une évolution dudit rapport (rEHV) sont mis en œuvre lorsque le rapport (rEHV) se situe en dessous ou au-dessus d'une valeur prédéterminée (ou qu'il se situe dans une plage de valeurs données). Par exemple, lorsque le rapport est compris entre -2,6 et -5, une donnée d'évolution du rapport peut être transmise car il est possible que cette valeur soit représentative d'un risque de chorioamniotite au cours de la rupture prématurée des membranes avant terme.

La valeur de référence (Vref), utilisée dans le calcul du rapport peut quant à elle être obtenue de plusieurs manières différentes. Selon une première approche, cette valeur de référence est issue d'une recherche effectuée dans un ou plusieurs atlas particuliers, recherche effectuée à partir de paramètres représentatifs du fœtus et/ou de la mère (poids estimé du fœtus, taille, nombre de semaines d'aménorrhée, poids et taille de la mère, antécédents, etc.). Cette recherche délivre une valeur de référence (Vref). Cette valeur de référence (Vref) est par exemple une moyenne du nombre d'épisodes de haute variation issue de constatations réalisées antérieurement sur un ensemble de cas correspondant aux paramètres représentatifs préalablement définis. Cette première approche permet de catégoriser un enregistrement cardiaque fœtal de manière très rapide. En effet, en utilisant une grande quantité de données préalablement obtenues à partir d'autres cas similaires (au niveau des paramètres choisis), il n'est pas nécessaire de disposer d'un historique de l'état du fœtus pour mettre en œuvre la technique proposée.

Selon une deuxième approche, cette valeur de référence (Vref) est obtenue en tenant compte d'enregistrements préalablement réalisés pour le fœtus. Par exemple, une valeur de référence (Vref) est calculée comme étant la moyenne du nombre d'épisodes de haute variation issue d'un nombre donné d'enregistrements précédemment réalisés (par exemple 4).On calcule ainsi une valeur de référence (Vref) qui est la moyenne du nombre d'épisodes de haute variation des enregistrements précédents. Cette moyenne est fortement corrélée au statut fœtal. Elle est ainsi adaptée à une comparaison dans le cadre de la technique proposée.

Selon une troisième approche, plus simple à mettre en œuvre, cette valeur de référence (Vref) est définie en fonction du nombre de semaines d'aménorrhée ou du nombre de semaines grossesse. Il s'agit alors d'une valeur moyenne uniformément applicable.

Par ailleurs, le module de calcul comprend également par exemple :

- des moyens de calcul d'une donnée représentative d'un rapport entre la valeur représentative d'un nombre d'épisodes de haute variation (vNbHV) et la une valeur représentative d'un nombre d'épisodes de basse variation (vNbBV), le rapport étant appelé rapport des épisodes de variation (rEV) ;
- des moyens de calcul d'une donnée représentative d'une variation à court terme (vVCT) dudit rythme cardiaque foetal ;
- des moyens de calcul d'une donnée représentative d'un rapport entre la valeur représentative d'une variation à court terme (vVCT) et au moins une valeur de référence (VRefCt), le rapport étant appelé rapport des variations à court terme (rVCT) ;

La valeur de référence (VRefCt), utilisée dans le calcul du rapport des variations à court terme (rVCT) peut être obtenue de plusieurs manières différentes. Selon une première approche, cette valeur de référence (VRefCt) est issue d'une recherche effectuée dans un ou plusieurs atlas particuliers, recherche effectuée à partir de paramètres représentatifs du fœtus et/ou de la mère (poids estimé du fœtus, taille, nombre de semaines d'aménorrhée, poids et taille de la mère, antécédents, etc.). Cette recherche délivre une valeur de référence (VRefCt). Cette valeur de référence (VRefCt) est par exemple une moyenne des variations à court terme (vVCT) issue de constatations réalisée antérieurement sur un ensemble de cas correspondant aux paramètres représentatifs préalablement définis. Cette première approche permet de catégoriser un enregistrement cardiaque fœtal de manière très rapide. En effet, en utilisant une grande quantité de données préalablement obtenues à partir d'autres cas similaires (au niveau des paramètres choisis), il n'est pas nécessaire de disposer d'un historique de l'état du fœtus pour mettre en œuvre la technique proposée.

Selon une deuxième approche, cette valeur de référence (VRefCt) est obtenue en tenant compte d'enregistrements préalablement réalisés pour le fœtus. Par exemple, une valeur de référence est calculée comme étant la moyenne du nombre d'épisodes de haute variation issue d'un nombre donné d'enregistrements précédemment réalisés (par exemple 4).On calcule ainsi une valeur de référence qui est la moyenne des épisodes de haute variation des enregistrements précédents. Cette moyenne est fortement corrélée à la situation du fœtus. Elle est ainsi adaptée à une comparaison dans le cadre de la technique proposée.

Selon une troisième approche, plus simple à mettre en œuvre, cette valeur de référence est définie en fonction du nombre de semaines d'aménorrhée ou du nombre de semaines grossesse. Il s'agit alors d'une valeur moyenne uniformément applicable.

Le module de calcul (M-CALC) comprend également des moyens de détermination et d'obtention d'au moins certaines caractéristiques de la *variabilité* cardiaque. La *variabilité* du rythme cardiaque vise à approcher de façon non invasive la régulation du nœud sino-atrial par les branches sympathiques et parasympathiques du système nerveux autonome. La *variabilité* du rythme cardiaque est basée sur le principe selon lequel le rythme cardiaque n'évolue pas d'une manière constante au cours du temps et qu'il existe une fluctuation battement par battement autour d'une valeur moyenne. L'analyse de la *variabilité* du rythme cardiaque permet, d'une manière générale, d'obtenir des indices reflétant l'activité du système autonome. Le module d'analyse comprend ainsi les sous modules d'analyse de la variabilité du rythme cardiaque fœtal suivants :

- sous module d'analyse linéaire comprenant :

  ○ un composant d'analyse dans le domaine temporel : basé sur la mesure « battement à battement » (intervalle RR dans le cas de l'ECG) dénommée « NN » (pour « normal to normal ») :

    ▪ SDNN/SD (Standard deviation of NN intervals): Déviation standard de l'intervalle RR sur toute la période d'enregistrement; renseigne sur la variabilité globale ;
    ▪ rMSSD (root mean square of successive differences) : Racine carrée des différences au carré des intervalles RR successifs : exprime la variabilité de haute fréquence (principalement d'origine parasympathique) ;

  ○ un composant d'analyse dans le domaine fréquentiel (réalisant une analyse spectrale) d'étude de la distribution des ondes en fonction de leur fréquence. Les résultats présentent les éléments suivants :

    ▪ Les hautes fréquences (HF) : entre 0,15 et 0,4 Hz ; indicateur de l'activité parasympathique
    ▪ Les basses fréquences (LF) : entre 0,04 et 0,15 Hz ; relié au baroréflexe, indicateur de l'activité sympathique
    ▪ Les très basses fréquences (VLF) : entre 0,0033 et 0,04 Hz ; indicateur des mécanismes de régulation à long terme
    ▪ Les ultrabasses fréquences (ULF) : entre 0 et 0,0033 Hz

- un sous module d'analyse non linéaire (basé sur la théorie du chaos) dont la fonction est de déceler le déterminisme du signal et à en évaluer la complexité. Il comprend :

  ○ un composant entropique : entropie approximée (ApEn) et entropie échantillonnée (SampEn). Ces deux grandeurs sont des mesures de complexité et peuvent être vues comme des indicateurs de la régularité de la série RR observée
  ○ un composant de calcul de DFA (« *Detrended Fluctuation Analysis* ») et de ses coefficients caractéristiques ($\alpha 1$, $\alpha 2$). Ces coefficients mesurent le niveau d'organisation d'une série R- R entre 0,5 (totalement aléatoire) et 1,5 (totalement prédictible car suit un cycle fixe). Ils quantifient la corrélation court et long terme de séries temporelles stationnaires ou non.
  ○ un composant de calcul de Diagramme de Poincaré : il s'agit d'une représentation graphique des intervalles RR en fonction du précédent (corrélation entre intervalles successifs) ; peut être analysé qualitativement (forme du diagramme) et quantitativement :

    ▪ SD1 (standard deviation 1) est l'indice de variabilité à court terme, reflet de l'activité parasympathique sur le nœud sinusal.
    ▪ SD2 (standard deviation 2) est l'indice de variabilité à long terme et reflète à la fois l'activité sympathique et parasympathique sur le nœud sinusal.

  ○ un composant de calcul de dimension de corrélation : dans une représentation bidimensionnelle, l'axes de x représente la fréquence X[n] tandis que l'axe des y représente la fréquence X[x+retard]. Le choix d'un retard approprié est réalisé en utilisant la technique de calcul de l'information minimum mutuelle ; La propagation de la parcelle de l'espace des phases est différente pour diverses anomalie cardiaques ;

∘ un composant de calcul de l'exposant de Hurst: l'Exposant de Hurst est utilisé pour évaluer la présence ou l'absence de dépendance à long terme et le degré de cette dépendance dans une série temporelle ;

∘ un composant de calcul de dimension fractale : La notion de dimension fractale (FD) fait référence à un non entier et à des dimensions fractionnaires provient de la géométrie fractale. La FD est utilisée pour fournir une mesure de combien d'espace un objet occupe entre des dimensions euclidiennes ;

∘ un composant de calcul de l'exposant de Lyapunov : L'exposant de Lyapunov (k) est une mesure quantitative de la dépendance sensible aux conditions initiales. Il définit le taux moyen de divergence de deux trajectoires voisines.

∘ un composant de calcul de l'analyse de complexité de Lempel-Ziv.

La complexité de Lempel-Ziv (CLZ) pour les séquences finies a été proposée comme une mesure non paramétrique de la complexité des signaux. La CLZ exprime le nombre de sous-chaînes distinctes et leur récurrence dans une séquence. Une grande valeur signifie des données plus complexes. La CLZ peut-être, par conséquent, un outil utile pour quantifier la maturité et est donc adaptée au traitement des données du signal de fréquence cardiaque de l'invention.

Pour calculer la complexité de LZ, le signal analysé doit d'abord subir une quantification et en particulier être transformé en suite de 0, 1 tel que :

Si P=(s(1),s(2),...,s(n))
s(i)=0 si x(i)<T
s(i)=1 si x(i)>T
où x(i) est le signal

Par exemple, étant donnée une séquence binaire S = s1s2 ... sn, avec si {0,1} et i = 1, ..., n, on recherche dans le signal des mots distincts qui seront encodées. Il existe plusieurs manières pour effectuer l'analyse. Pour plus de simplicité, on propose l'approche proposée dans la littérature. Par exemple, après l'analyse de la séquence 10101110100 devient 1.0.10.11.101.00 et le nombre de mots, noté c (n), est de 6.

$$C_{LZ} = \frac{c(n)}{n / \log_2 n}$$

- La CLZ devient : où n est la longueur de la séquence étudiée.

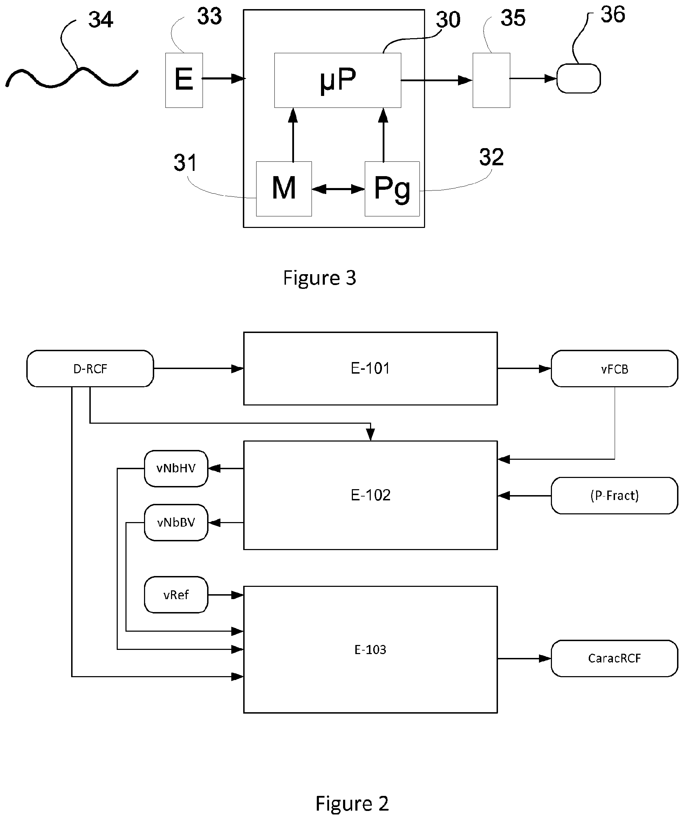

La figure 2 se rapporte à une méthode implémentée dans le dispositif préalablement décrit. Plus particulièrement, selon un mode de réalisation, l'invention se rapporte à une méthode de traitement de données de rythme cardiaque fœtal (D-RCF) issue d'un enregistrement de rythme cardiaque fœtal, données obtenues par l'intermédiaire d'un dispositif d'enregistrement de rythme cardiaque fœtal comprenant :

- une étape de détermination (E-101) d'une fréquence cardiaque de référence, prenant en entrée les données de rythme cardiaque fœtal (D-RCF) et délivrant une valeur de fréquence cardiaque de référence (vFCB);

- une étape de fractionnement (E-102) desdites données, en fonction d'un paramètre de fractionnement (P-Fract) et de la valeur de fréquence cardiaque de référence (vFCB) ;

- une étape de calcul (E-103) d'au moins une caractéristique (CaracRCF) représentative des données de rythme cardiaque fœtal (D-RCF) en fonction de la valeur de fréquence cardiaque de référence (vFCB).

5.2. Description d'un mode de réalisation

Dans un premier exemple, la technique décrite peut être complétée par d'autres types de données cliniques. Par exemple, un système basé sur l'analyse quantitative informatisée des signaux biophysiques fœtaux (cardiaques, respiratoires, mouvements actifs entre autres) au cours de ses différents états comportementaux couplé à la méthode et au dispositif d'analyse proposé peut être mis en œuvre. Un tel système permettrait d'informer les praticiens sur le statut fœtal et/ou l'issue de grossesse et/ou une condition pathologique de la grossesse.

Un tel système comprend des moyens de détection de différents signaux biophysiques fœtaux et leur analyse afin de révéler des changements d'états comportementaux du fœtus (sommeil actif, sommeil calme, éveil actif et éveil calme). Les changements observés dans l'évolution des signaux biophysiques recueillis en fonction des changements d'états comportementaux définissent alors des marqueurs d'intérêt. L'expression quantitative ou modélisée de l'évolution de ces marqueurs permet de définir des indicateurs de surveillance lors de la grossesse et lors de pathologies foeto-placentaires. L'avantage de comparer l'évolution des signaux biophysiques recueillis en fonction des changements d'états comportementaux est d'obtenir un résultat en valeur relative plutôt qu'en valeur absolue (soumise à des variations potentiellement importantes et multifactorielles).

La technique décrite peut être mise en œuvre tant dans l'obtention d'indicateurs antepartum que dans l'obtention d'indicateurs intrapartum.

En antepartum, les centres d'intérêts sont particulièrement :

- évaluer le bien être fœtal en cas de restriction de croissance
- différencier la restriction de croissance du petit poids pour l'âge gestationnel
- évaluer le risque de chorioamniotite et/ou d'infection materno-fœtale en cas de rupture prématurée des membranes avant terme ou à terme
- évaluer le risque d'accouchement prématuré
- évaluer la maturation physiologique fœtale à terme et avant terme

En intrapartum, les centres d'intérêts sont particulièrement :

- évaluation du risque d'anoxie/asphyxie fœtale au cours du travail
- évaluation du risque d'infection materno-fœtale
- évaluation du risque d'encéphalopathie

Un autre exemple d'un type d'un module d'analyse est un détecteur de condition clinique. Très généralement, un détecteur de condition clinique comprend un extracteur de caractéristiques pour extraire des caractéristiques des données de l'enregistrement cardiaque fœtal, tels que la fréquence cardiaque de référence, le nombre d'accélérations des battements cardiaques, le nombre d'épisodes de haute variation des battements cardiaques. Ces caractéristiques sont alors fournies à un évaluateur d'état clinique, qui identifie des modèles d'enregistrements spécifiques (à partir d'un atlas par exemple) qui sont corrélés avec les événements d'importance clinique.

Par exemple, l'évaluateur d'état clinique peut utiliser un modèle pour corréler les comportements électro-physiologiques du fœtus et/ou de la mère à des comportements statistiques associés à des conditions médicales pré-identifiées, comme par exemple la chorioamniotite dans les ruptures prématurées des membranes avant terme ou l'infection néonatale clinique. La corrélation obtenue est utilisée pour déterminer la susceptibilité du patient (la mère et/ou le fœtus) de se trouver dans une telle situation.

Selon le mode de réalisation particulier, l'évaluateur d'état clinique peut avoir des modules distincts (par exemple, un évaluateur de Chorioamniotite, un évaluateur de la fièvre intra-partum, etc.); chaque module fournissant une mesure distincte en lien avec cet évaluateur. Les praticiens peuvent recevoir les données de ces différents modules dans les scores de confiance, par exemple, présenté sur une échelle de 0 à 10 avec "0" indiquant une probabilité d'absence (ou moins) d'un état et "10" indiquant une probabilité de présence d'un état. Les scores de confiance individuels peuvent également être combinés pour former une évaluation fœtale globale.

### 5.3. Description d'un deuxième mode de réalisation

Dans ce deuxième mode de réalisation, la technique décrite met en œuvre une analyse segmentée. Plus particulièrement, le module de calcul (M-CALC) met en œuvre les moyens décrits précédemment :

- uniquement sur les épisodes de basse variation (sommeil calme) ; et/ou
- uniquement sur les épisodes de haute variation (sommeil agité) ; et/ou
- en calculant un rapport de ces caractéristiques sur chaque type d'épisode.

Pour ce faire, les données fractionnées sont regroupées. Un traitement particulier est effectué par le module de fractionnement (M-Fct) desdites données, en fonction du paramètre de fractionnement (P-Fract) et de la valeur de fréquence cardiaque de référence (vFCB) comme explicité préalablement. Auquel cas, les calculs effectués postérieurement à la phase de fractionnement peuvent se limiter aux épisodes de basse variation ou aux épisodes de haute variation par exemple.

Dans ce deuxième mode de réalisation le module de calcul (M-CALC) peut en outre utiliser des données représentatives d'un état comportemental du fœtus en sus des données de variabilité du rythme cardiaque fœtal. Ainsi, le module d'analyse disposant des données relatives à des changements d'états comportementaux du fœtus au cours de l'enregistrement peut calculer une corrélation entre les changements comportementaux du fœtus et les données du rythme cardiaque fœtal. Dès lors, le résultat obtenu par le module d'analyse est un résultat relatif. Un tel résultat est avantageux puisqu'il permet de contextualiser des variations du rythme cardiaque fœtal.

### 5.4. Description d'un troisième mode de réalisation

Une étude menée sur plusieurs patientes a montré que la rapport du nombre d'épisodes de haute variation sur le nombre d'épisodes de basse variation pouvait être significatif d'une infection de type Chorioamniotite. De même, elle a montré une corrélation entre la valeur de la VCT et le risque d'infection.

### 5.5. Autres caractéristiques et avantages

On décrit, en relation avec la figure 3, un dispositif, tel qu'un moniteur, mis en œuvre pour analyser un enregistrement de rythme cardiaque fœtal.

Par exemple, le dispositif comprend une mémoire 31 constituée d'une mémoire tampon, une unité de traitement 32, équipée par exemple d'un microprocesseur, et pilotée par le programme d'ordinateur 33, mettant en œuvre un procédé d'analyse.

À l'initialisation, les instructions de code du programme d'ordinateur 33 sont par exemple chargées dans une mémoire avant d'être exécutées par le processeur de l'unité de traitement 32. L'unité de traitement 32 reçoit en entrée au moins une donnée représentative d'un rythme cardiaque fœtal, issue d'un enregistrement en temps réel ou en temps différé. Le microprocesseur de l'unité de traitement 32 met en œuvre les étapes du procédé d'analyse, selon les instructions du programme d'ordinateur 33 pour calculer au moins une fréquence cardiaque de référence, des épisodes de haute ou basse variation de fréquence cardiaque, etc);

Pour cela, le moniteur comprend, outre la mémoire tampon 31, des moyens de communications, tels que des modules de communication réseau, des moyens de transmission de donnée, des moyens de captation des données représentatives du rythme cardiaque fœtal.

Ces moyens peuvent se présenter sous la forme d'un processeur particulier implémenté au sein du dispositif, le processeur étant un processeur sécurisé ou redondant. Selon un mode de réalisation particulier, ce dispositif met en œuvre une application particulière qui est en charge du traitement des données issues du rythme cardiaque fœtal afin de délivrer les valeurs susmentionnées.

Par ailleurs, le dispositif comprend en outre des moyens d'obtention de valeurs de référence. Ces moyens se présentent également comme des interfaces de communications permettant d'échanger des données sur des réseaux de communication, des moyens d'interrogations et de mise à jour de base de données.

**Revendications**

1. Dispositif de traitement de données de rythme cardiaque foetal (D-RCF) issue d'un enregistrement de rythme cardiaque foetal, données obtenues par l'intermédiaire d'un dispositif d'enregistrement de rythme cardiaque foetal, dispositif comprenant :

    - un module de détermination (MD-FCB) d'une fréquence cardiaque de référence, prenant en entrée les données de rythme cardiaque foetal (D-RCF) et délivrant une valeur de fréquence cardiaque de référence (vFCB);
    - un module de fractionnement (M-Fct) desdites données, en fonction d'un paramètre de fractionnement (P-Fract) et de la valeur de fréquence cardiaque de référence (vFCB) ; ledit module de fractionnement (M-Fct) comprenant

        ◦ des moyens de détermination d'un nombre d'épisodes de haute variation des battements cardiaques, les moyens de détermination prenant en entrée les données constitutives de l'enregistrement du rythme cardiaque foetal et délivrant au moins une valeur représentative d'un nombre d'épisodes de haute variation (vNbHV) ;

        ◦ des moyens de détermination d'un nombre d'épisodes de basse variation des battements cardiaques, les moyens de détermination prenant en entrée les données constitutive de l'enregistrement du rythme cardiaque foetal et délivrant au moins une valeur représentative d'un nombre d'épisodes de basse variation (vNbBV) ;

    - un module de calcul (M-CALC) d'au moins une caractéristique représentative des données de rythme cardiaque foetal (D-RCF) en fonction de la valeur de fréquence cardiaque de référence (vFCB) ;

    le dispositif est **caractérisé en ce que** le module de calcul comprend des moyens de calcul d'un rapport entre le nombre d'épisodes de haute variation (vNb-HV) et le nombre d'épisodes de basse variation (vNbBV), le rapport étant appelé rapport des épisodes de variation (rEV);

2. Dispositif selon la revendication 1, **caractérisé en ce que** le module de fractionnement (M-Fct) comprend :

    - des moyens de détermination d'un nombre d'accélérations et de décélérations des battements cardiaques, les moyens de détermination prenant en entrée les données constitutives de l'enregistrement du rythme cardiaque fœtal et la valeur de fréquence cardiaque de référence (vFCB) et délivrant au moins une valeur représentative d'un nombre d'accélérations .

3. Dispositif selon la revendication 1, **caractérisé en ce que** le module de calcul (M-CALC) d'au moins une caractéristique (CaracRCF) représentative des données de rythme cardiaque foetal (D-RCF) en fonction de la valeur de fréquence cardiaque de référence (vFCB) comprend :

    - des moyens de calcul d'une donnée représentative d'un rapport entre la valeur représentative d'un nombre d'épisodes de haute variation (vNbHV) et au moins une valeur de référence (VRef), le rapport étant appelé rapport des épisodes de haute variation (rEHV) ;
    - des moyens de transmission d'une donnée représentative d'une évolution dudit rapport des épisodes de haute variation (rEHV).

4. Dispositif selon la revendication 1, **caractérisé en ce que** le module de calcul (M-CALC) d'au moins une caractéristique (CaracRCF) représentative des données de rythme cardiaque foetal (D-RCF) en fonction de la valeur de fréquence cardiaque de référence (vFCB) comprend :

- des moyens de calcul d'une donnée représentative d'une variation à court terme (vVCT) dudit rythme cardiaque foetal ;
- des moyens de calcul d'une donnée représentative d'un rapport entre la valeur représentative d'une variation à court terme (vVCT) et au moins une valeur de référence (VRefCt), le rapport étant appelé rapport des variations à court terme (rVCT).

5. Dispositif selon la revendication 1, **caractérisé en ce que** le module de calcul (M-CALC) d'au moins une caractéristique représentative les données de rythme cardiaque foetal (D-RCF) en fonction de la valeur de fréquence cardiaque de référence (vFCB) comprend un sous module d'analyse linéaire comprenant :

- un composant d'analyse dans le domaine temporel basé sur une mesure « battement à battement » dénommée « NN » (pour « normal to normal ») :
- un composant d'analyse dans le domaine fréquentiel, réalisant une analyse spectrale d'étude de la distribution des ondes en fonction de leur fréquence.

6. Dispositif selon la revendication 1, **caractérisé en ce que** le module de calcul (M-CALC) d'au moins une caractéristique représentative les données de rythme cardiaque foetal (D-RCF) en fonction de la valeur de fréquence cardiaque de référence (vFCB) comprend un sous module d'analyse non linéaire comprenant :

- un composant entropique d'obtention d'une entropie approximée (ApEn) et d'une entropie échantillonnée (SampEn).
- un composant de calcul de DFA (« Detrended Fluctuation Analysis ») et de ses coefficients caractéristiques ($\alpha1$, $\alpha2$)
- un composant de calcul de Diagramme de Poincaré.

7. Méthode de traitement de données de rythme cardiaque foetal (D-RCF) issue d'un enregistrement de rythme cardiaque foetal, données obtenues par l'intermédiaire d'un dispositif d'enregistrement de rythme cardiaque foetal, ladite méthode est mise en œuvre par un dispositif selon la revendication 1 et comprend:

- une étape de détermination (E-101) d'une fréquence cardiaque de référence, prenant en entrée les données de rythme cardiaque foetal (D-RCF) et délivrant une valeur de fréquence cardiaque de référence (vFCB);
- une étape de fractionnement (E-102) desdites données, en fonction d'un paramètre de fractionnement (P-Fract) et de la valeur de fréquence cardiaque de référence (vFCB), l'étape de fractionnement comprenant :

- une sous-étape de détermination d'un nombre d'épisodes de haute variation des battements cardiaques prenant en entrée les données constitutives de l'enregistrement du rythme cardiaque foetal et délivrant au moins une valeur représentative d'un nombre d'épisodes de haute variation (vNbHV) ;
- une sous-étape de détermination d'un nombre d'épisodes de basse variation des battements cardiaques prenant en entrée les données constitutives de l'enregistrement du rythme cardiaque foetal et délivrant au moins une valeur représentative d'un nombre d'épisodes de basse variation (vNbBV)

- une étape de calcul (E-103) d'au moins une caractéristique (CaracRCF) représentative des données de rythme cardiaque foetal (D-RCF) en fonction de la valeur de fréquence cardiaque de référence (vFCB) ;

la méthode est **caractérisée en ce que** l'étape de calcul comprend le calcul du rapport entre la valeur le nombre d'épisodes de haute variation (vNbHV) et le nombre d'épisodes de basse variation (vNbBV), le rapport étant appelé rapport des épisodes de variation (rEV).

8. Produit programme d'ordinateur téléchargeable depuis un réseau de communication et/ou stocké sur un support lisible par ordinateur et/ou exécutable par un microprocesseur, **caractérisé en ce qu'**il comprend des instructions de code de programme pour l'exécution d'un procédé de traitement de données selon la revendication 7, lorsqu'il est exécuté sur un ordinateur.

**Patentansprüche**

1. Vorrichtung zum Verarbeiten von fetalen Herzfrequenzdaten (D-RCF) aus einer Aufzeichnung der fetalen Herzfrequenz, wobei die Daten durch eine Vorrichtung zum Aufzeichnen der fetalen Herzfrequenz erhalten werden, die Vorrichtung umfassend:

- ein Modul zum Bestimmen (MD-FCB) einer Referenzherzfrequenz, bei dem die fetalen Herzfrequenzdaten (D-RCF) eingegeben werden und ein Referenz-Herzfrequenzwert (vFCB) bereitgestellt wird,

- ein Fraktionierungsmodul (M-Fct) der Daten in Abhängigkeit von einem Fraktionierungsparameter (P-Fract) und des Referenz-Herzfrequenzwerts (vFCB), wobei das Fraktionierungsmodul (M-Fct) aufweist

    o Mittel zum Bestimmen einer Anzahl von Episoden hoher Variation der Herzschläge, wobei die Mittel zum Bestimmen als Eingabe die Daten nehmen, die die Aufzeichnung der fetalen Herzfrequenz bilden und mindestens einen Wert bereitstellen, der für eine Anzahl von Episoden hoher Variation (vNbHV) repräsentativ ist,
    o Mittel zum Bestimmen einer Anzahl von Episoden niedriger Variation der Herzschläge, wobei die Mittel zum Bestimmen als Eingabe die Daten nehmen, die die Aufzeichnung der fetalen Herzfrequenz bilden und mindestens einen Wert bereitstellen, der für eine Anzahl von Episoden niederer Variation (vNbBV) repräsentativ ist,

- ein Modul zum Berechnen (M-CALC) mindestens eines Merkmals, das für die fetalen Herzfrequenzdaten (D-RCF) repräsentativ ist, in Abhängigkeit von dem Referenz-Herzfrequenzwert (vFCB),

wobei die Vorrichtung **dadurch gekennzeichnet ist, dass** das Modul zum Berechnen Mittel zum Berechnen eines Verhältnisses zwischen der Anzahl von Episoden hoher Variation (vNbHV) und der Anzahl von Episoden niedriger Variation (vNbBV) aufweist, wobei das Verhältnis, Verhältnis der Variationsepisoden (rEV) genannt wird.

2. Vorrichtung nach Anspruch 1, **dadurch gekennzeichnet, dass** das Fraktionierungsmodul (M-Fct) aufweist:

- Mittel zum Bestimmen einer Anzahl von Beschleunigungen und Verzögerungen der Herzschläge, wobei die Mittel zum Bestimmen als Eingabe die Daten nehmen, die die Aufzeichnung der fetalen Herzfrequenz * und den Referenz-Herzfrequenzwert (vFCB) bilden und mindestens einen Wert bereitstellen, der für eine Anzahl von Beschleunigungen repräsentativ ist.

3. Vorrichtung nach Anspruch 1, **dadurch gekennzeichnet, dass** das Modul zum Berechnen (M-CALC) mindestens eines Merkmals (CaracRCF), das für die fetalen Herzfrequenzdaten (D-RCF) repräsentativ ist, in Abhängigkeit von dem Referenz-Herzfrequenzwert (vFCB) aufweist:

- Mittel zum Berechnen einer Dateneinheit, die für ein Verhältnis zwischen einer Anzahl von Episoden hoher Variation (vNbHV) und mindestens einem Referenzwert (VRef) repräsentativ ist, wobei das Verhältnis Verhältnis der Episoden hoher Variation (rEHV) genannt wird,
- Mittel zum Übertragen einer Dateneinheit, die für eine Entwicklung des Verhältnisses der Episoden hoher Variation (rEHV) repräsentativ ist.

4. Vorrichtung nach Anspruch 1, **dadurch gekennzeichnet, dass** das Modul zum Berechnen (M-CALC) mindestens eines Merkmals (CaracRCF), das für die fetalen Herzfrequenzdaten (D-RCF) repräsentativ ist, in Abhängigkeit von dem Referenz-Herzfrequenzwert (vFCB) aufweist:

- Mittel zum Berechnen einer Dateneinheit, die für eine kurzfristige Variation (vVCT) der fetalen Herzfrequenz repräsentativ ist,
- Mittel zum Berechnen einer Dateneinheit, die für ein Verhältnis zwischen einer kurzfristigen Variation (vVCT) und mindestens einem Referenzwert (VRefCt) repräsentativ ist, wobei das Verhältnis, Verhältnis der kurzfristigen Variationen (rVCT) genannt wird.

5. Vorrichtung nach Anspruch 1, **dadurch gekennzeichnet, dass** das Modul zum Berechnen (M-CALC) mindestens eines Merkmals, das für die fetalen Herzfrequenzdaten (D-RCF) repräsentativ ist, in Abhängigkeit von dem Referenz-Herzfrequenzwert (vFCB) ein Untermodul der linearen Analyse aufweist:

- eine Analysekomponente im Zeitbereich, die auf einer "Schlag zu Schlag"-Messung basiert, die "NN" (für "normal to normal") genannt wird;
- eine Analysekomponente im Frequenzbereich, die eine Spektralanalyse zur Untersuchung der Verteilung der Wellen in Abhängigkeit von ihrer Frequenz durchführt.

6. Vorrichtung nach Anspruch 1, **dadurch gekennzeichnet, dass** das Modul zum Berechnen (M-CALC) mindestens eines Merkmals, das für die fetalen Herzfrequenzdaten (D-RCF) repräsentativ ist, in Abhängigkeit von dem Referenz-Herzfrequenzwert (vFCB) ein Untermodul der nichtlinearen Analyse aufweist:

- eine Entropiekomponente zum Erhalten einer angenäherten Entropie (A-pEn) und einer abgetasteten Entropie (SampEn);
- eine Komponente zum Berechnen der DFA ("Trendbereinigte Fluktuationsanalyse") und ihrer charakteristischen Koeffizienten ($\alpha 1$, $\alpha 2$);
- eine Komponente zum Berechnen des Poincare-Diagramms.

**7.** Verfahren zum Verarbeiten von fetalen Herzfrequenzdaten (D-RCF) aus einer Aufzeichnung der fetalen Herzfrequenz, Daten, die durch eine Vorrichtung zum Aufzeichnen der fetalen Herzfrequenz erhalten werden, wobei das Verfahren durch eine Vorrichtung nach Anspruch 1 umgesetzt wird und aufweist:

- einen Schritt des Bestimmens (E-101) einer Referenzherzfrequenz, bei dem die fetalen Herzfrequenzdaten (D-RCF) eingegeben werden und ein Referenz-Herzfrequenzwert (vFCB) bereitgestellt wird,
- einen Schritt des Fraktionierens (E-102) der Daten in Abhängigkeit von einem Fraktionierungsparameter (P-Fract) und des Referenz-Herzfrequenzwerts (vFCB), wobei der Schritt des Fraktionierens aufweist:

- einen Unterschritt des Bestimmens einer Anzahl von Episoden hoher Variation der Herzschläge, bei dem Daten eingegeben werden, die die Aufzeichnung der fetalen Herzfrequenz bilden, und mindestens ein Wert bereitgestellt wird, der für eine Anzahl von Episoden hoher Variation (vNbHV) repräsentativ ist,
- einen Unterschritt des Bestimmens einer Anzahl von Episoden niederer Variation der Herzschläge, in dem Daten eingegeben werden, die die Aufzeichnung der fetalen Herzfrequenz bilden, und mindestens ein Wert bereitgestellt wird, der für eine Anzahl von Episoden niederer Variation (vNbBV) repräsentativ ist,

- einen Schritt des Berechnens (E-103) mindestens eines Merkmals (CaracRCF), das für die fetalen Herzfrequenzdaten (D-RCF) repräsentativ ist, in Abhängigkeit von dem Referenz-Herzfrequenzwert (vFCB),

das Verfahren ist **dadurch gekennzeichnet, dass** der Schritt des Berechnens das Berechnen eines Verhältnisses zwischen der Anzahl von Episoden hoher Variation (vNbHV) und der Anzahl von Episoden niedriger Variation (vNbBV) aufweist, wobei das Verhältnis Verhältnis der Variationsepisoden (rEV) genannt wird.

**8.** Computerprogrammprodukt, das von einem Kommunikationsnetz heruntergeladen werden kann und/oder auf einem computerlesbaren Medium gespeichert ist und/oder von einem Mikroprozessor ausgeführt werden kann, **dadurch gekennzeichnet, dass** es Programmcodeanweisungen zum Ausführen eines Datenverarbeitungsverfahrens nach Anspruch 7 aufweist, wenn es von einem Computer ausgeführt wird.

**Claims**

**1.** Device for processing foetal heart rate data (D-RCF) from a foetal heart rate record, said data being obtained through a foetal heart rate recording device, said device comprising:

- a module (MD-FCB) for determination of a reference heart rate, taking as input the foetal heart rate data (D-RCF) and delivering a reference heart rate value (vFCB);
- a module (M-Fct) for fractionation of said data, as a function of a fractionation parameter (P-Fract) and the reference heart rate value (vFCB);

∘ means for determining a number of episodes of high variation of heart beats, the determining means taking as input the data constituting the recording of the foetal heart rate and delivering at least one value representative of a number of episodes of high variation (vNbHV);
∘ means for determining a number of episodes of low variation of heart beats, the determining means taking as input the data constituting the recording of the foetal heart rate and delivering at least one value representative of a number of episodes of low variation (vNbBV);

- a module (M-CALC) for calculation of at least one representative characteristic of the foetal heart rate data (D-RCF) as a function of the reference heart rate value (vFCB);

the device being **characterised in that** the calculation module comprises means for calculating a ratio between the number of episodes of high variation (vNbHV) and the number of episodes of low variation (vNbBV), the ratio being called ratio of episodes of variation (rEV).

**2.** Device according to claim 1, **characterised in that** the fractionation module (M-Fct) comprises:

- means for determining a number of accelerations and decelerations of the heart beats, the determining means taking as input the data constituting the recording of the foetal heart rate and the reference heart rate value (vFCB) and delivering at least one value representative of a number of accelerations.

**3.** Device according to claim 1, **characterised in that** the module (M-CALC) for calculation of at least one characteristic (CaracRCF) representative of the foetal heart rate data (D-RCF) as a function of the reference heart rate value (vFCB) includes:

- means for calculating an item of data representative of a ratio between the value representative of a number of episodes of high variation (vNbHV) and at least one reference value (VRef), the ratio being called a ratio of the episodes of high variation (rEHV);
- means for transmitting an item of data representative of an evolution of said ratio of high variation episodes (rEHV) .

4. Device according to claim 1, **characterised in that** the module (M-CALC) for calculation of at least one characteristic (CaracRCF) representative of the foetal heart rate data (D-RCF) as a function of the reference heart rate value (vFCB) comprises:

- means for calculating an item of data representative of a short-term variation (vVCT) of said foetal heart rate;
- means for calculating an item of data representative of a ratio between the representative value of a short-term variation (vVCT) and at least one reference value (VRefCt), the ratio being called a ratio of short-term variations (rVCT).

5. Device according to claim 1, **characterised in that** the module (M-CALC) for calculation of at least one characteristic representative of the foetal heart rate data (D-RCF) as a function of the reference heart rate value (vFCB) comprises a linear analysis sub-module comprising:

- a time domain analysis component based on a "beat-to-beat" measurement called "NN" (for "normal to normal");
- an analysis component in the frequency domain, performing a spectral analysis of the study of the wave distribution according to frequency.

6. Device according to claim 1, **characterised in that** the module (M-CALC) for calculation of at least one characteristic representative of the foetal heart rate data (D-RCF) as a function of the reference heart rate value (vFCB) comprises a nonlinear analysis sub-module comprising:

- an entropy component for obtaining an approximated entropy (ApEn) and a sampled entropy (SampEn);
- a component for calculation of DFA ("Detrended Fluctuation Analysis") and its characteristic coefficients ($\alpha$1, $\alpha$2);
- a Poincaré diagram calculation component.

7. Method of processing foetal heart rate data (D-RCF) from a foetal heart rate record, said data obtained through a foetal heart rate recording device, said method being implemented by a device according to claim 1 and comprising:

- a sub-step (E-101) of determining a reference heart rate, taking as input the foetal heart rate data (D-RCF) and delivering a reference heart rate value (vFCB);
- a sub-step (E-102) of fractionating said data, based on a fractionation parameter (P-Fract) and the reference heart rate value (vFCB), the fractionating step comprising:

- a substep for determining a number of episodes of high variation of heart beats taking as input the data constituting the recording of the foetal heart rate and delivering at least one value representative of a number of episodes high variation (vNbHV);
- a substep for determining a number of episodes of low variation of heart beats taking as input the data constituting the recording of the foetal heart rate and delivering at least one value representative of a number of episodes of low variation (vNbBV);

- a step (E-103) for calculation of at least one characteristic (CaracRCF) representative of the foetal heart rate data (D-RCF) as a function of the reference heart rate value (vFCB);

the method being **characterised in that** the calculation step comprises the calculation of the ratio between the number of episodes high variation (vNbHV) and the number of episodes low variation (vNbBV), the ratio being called ratio of episodes of variation (rEV)

8. Computer program product downloadable from a communication network and/or stored on a computer readable medium and/or executable by a microprocessor, **characterised in that** it comprises program code instructions for executing a method of data processing according to claim 7 when executed on a computer.

Figure 1

Figure 3

Figure 2

## RÉFÉRENCES CITÉES DANS LA DESCRIPTION

**Documents brevets cités dans la description**

- US 2004133115 A1

**Littérature non-brevet citée dans la description**

- **ANDREA BRAVI.** *Review and classification of variability analysis*

- **DAS S.** *A novel approach for extraction and analysis of variability of baseline*